# EUROPÄISCHE PATENTSCHRIFT

(11) **EP 0 608 688 B1**
(45) Veröffentlichungstag und Bekanntmachung des Hinweises auf die Patenterteilung: **22.04.1998**
(21) Anmeldenummer: 94100176.0
(22) Anmeldetag: 07.01.1994
(51) Int. Cl.: C07D 207/323

(54) **Verfahren zur Reinigung von Rohpyrrolen**
Process for the purification of crude pyrroles
Procédé pour la purification de pyrroles bruts

(30) Priorität: 23.01.1993 DE 4301776
(43) Veröffentlichungstag der Anmeldung: 03.08.1994
(73) Patentinhaber: BASF Aktiengesellschaft, 67063 Ludwigshafen (DE)
(72) Erfinder: Pfab, Peter, Dr., 67117 Limburgerhof (DE); Wahl, Peter, Dr., 68526 Ladenburg (DE); Franz, Dieter, Dr., 67069 Ludwigshafen (DE)

(56) Entgegenhaltungen:
- EP-A- 0 067 360
- GB-A- 515 865
- GB-A- 852 130
- US-A- 3 555 021
- Ber. Dtsch.Chem.Ges.,27(1894),476-480

## Beschreibung

Die vorliegende Erfindung betrifft ein Verfahren zur Reinigung von bei der Herstellung von Pyrrolen der allgemeinen Formel I 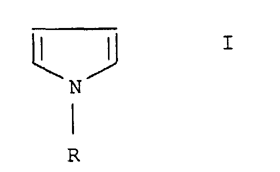 in der R Wasserstoff oder einen C₁-C₆-Alkylrest bedeutet, anfallenden Rohpyrrolen.

Pyrrole und Pyrrolderivate können nach verschiedenen Verfahren hergestellt werden (s. z.B. Ullmanns Encyklopädie der technischen Chemie, 4. Aufl. Bd. 19, S. 639-642). Eine Möglichkeit besteht darin, Pyrrolidine in Gegenwart bestimmter Katalysatoren zu dehydrieren (s. z.B. GB 515 865 und EP 67 360 B1).

Eine Schwierigkeit bei der Herstellung von Pyrrolen liegt darin, das Pyrrol nach der Reaktion in Reinform zu isolieren. Gemäß den EP 67 360 B1 wird dazu destillativ aufgearbeitet, was jedoch oft nicht zu ausreichend reinen Produkten führt. Aus der GB 515 865 ist es bekannt, N-Phenyl-pyrrol mit verdünnter HCl zu waschen und anschließend zu sublimieren. Die Sublimation des festen N-Phenyl-pyrrols ist jedoch aufwendig.

Von Bedeutung ist dabei insbesondere die möglichst vollständige Abtrennung von den Pyrrolidinen, was aufgrund der sich bildenden Azeotrope zwischen Pyrrol, Pyrrolidin und Wasser besonders schwierig ist.

Aufgabe der vorliegenden Erfindung war es daher, ein Verfahren zur Reinigung von Rohpyrrol zur Verfügung zu stellen, das zu hochreinen Produkten führt und verfahrenstechnisch einfach auszuführen ist.

Diese Aufgabe wird durch ein Verfahren zur Reinigung von bei der Herstellung von Pyrrolen der allgemeinen Formel I 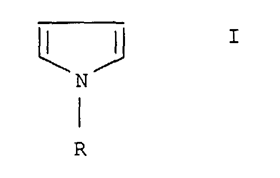 in der R Wasserstoff oder einen C₁-C₆-Alkylrest bedeutet, anfallenden Rohpyrrolen gelöst, das dadurch gekennzeichnet ist, daß man die Mischung, die das Rohpyrrol enthält, mit einer Säure oder wenn R Wasserstoff bedeutet, mit einer Säure oder einem aktivierten Carbonsäurederivat versetzt und anschließend das Pyrrol aus der Mischung bei Unterdruck und einer Sumpftemperatur bis zu 160°C destillativ abtrennt.

Es zeigt sich, daß durch die Kombination der Umsetzung mit einer Säure oder einem aktivierten Carbonsäurederivat und anschließender destillativer Aufarbeitung Pyrrole mit einem Restwassergehalt von unter 0,2 % und einem Pyrrolidingehalt von unter 0,3 % erhalten werden können.

Wenn erreicht werden soll, daß der Pyrrolidingehalt besonders niedrig ist, ist es bevorzugt, daß man die Rohpyrrol enthaltende Mischung mit einer mindestens 10 Gew.-%igen Mineral- oder Carbonsäure versetzt und das Pyrrol aus der Mischung bei 300 mbar bis 20 mbar und einer Sumpftemperatur von 60°C bis 90°C destillativ abtrennt.

Entscheidend ist dabei, daß die Sumpftemperatur in dem angegebenen Bereich eingestellt wird, ansonsten treten erhöhte Menge an Verunreinigungen auf.

Als Mineralsäure wird insbesondere Schwefelsäure, als Carbonsäure Ameisensäure, bevorzugt 60 bis 95 gew.-%ige Ameisensäure, eingesetzt. Soll das durch die Umsetzung mit Säure abgetrennte Pyrrolidin wieder zurückgewonnen werden, so geht man zweckmäßigerweise so vor, daß man die Rohpyrrol enthaltende Mischung mit einer 10 bis 30 Gew.-%igen Mineral- oder Carbonsäure versetzt und die sich bildende wäßrige Phase vor der destillativen Aufarbeitung des Pyrrols abtrennt. Aus der wäßrigen Phase läßt sich das Pyrrolidin nach entsprechender pH-Wert-Einstellung wiedergewinnen.

Wenn erreicht werden soll, daß der Wassergehalt in Pyrrol besonders niedrig ist, ist es bevorzugt, daß man die Rohpyrrol enthaltende Mischung unter Erwärmen mit einem aktivierten Carbonsäurederivat unter Bildung von Amiden versetzt und das Pyrrol aus der Mischung bei Drücken von 1000 mbar bis 200 mbar und Sumpftemperaturen von 80°C bis 160°C destillativ abtrennt. Bei dieser Verfahrensweise kann ein Wassergehalt im Reinpyrrol unter 0,1 % erreicht werden.

Als aktivierte Carbonsäurederivate kommen z.B. Carbonsäureanhydride, Carbonsäurechloride oder Carbonsäureester in Betracht. Bevorzugt sind Methyl- oder Ethylester von C₁-C₃-Monocarbonsäuren, insbesondere Methylformiat.

Bei der nach der Umsetzung mit Säure bzw. aktiviertem Carbonsäurederivaten stattfindenden destillativen Aufarbeitung geht zunächst eine geringe Menge Vorlauf über, der in das Rohpyrrol zurückgeführt werden kann.

Zweckmäßig ist es, das Rohpyrrol vor der Umsetzung mit der Säure oder dem aktivierten Carbonsäurederivat einer Vordestillation zu unterwerfen. Dies läßt sich z.B. so durchführen, daß man bei Unterdruck bis zu 40 mbar und einer Sumpftemperatur bis zu 85°C die Pyrrole von dem Sumpfrückstand abtrennt. Der Vorlauf bei dieser. Vordestillation enthält etwa 80 % Pyrrol, 15 % Pyrrolidin und 3 % Wasser und kann in das Rohpyrrol zurückgeführt werden, die wasserhelle Hauptfraktion enthält etwa 95 Gew.-% des ursprünglich vorhandenen Pyrrols.

Besonders gut geeignet ist das vorliegende Reinigungsverfahren für Rohpyrrole, die man dadurch herstellt, daß man Pyrrolidine der Formel II 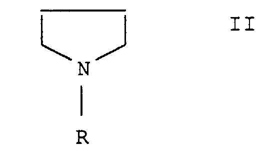 worin R die im Anspruch 1 angegebene Bedeutung hat, bei Temperaturen von 160°C bis 400°C in Gegenwart von Palladiumträgerkatalysatoren, die basische Verbindungen der Alkali-, Erdalkalimetalle und/oder Metalle der seltenen Erden und/ oder Elemente der Gruppen Ib, IIb, VIIb, Cobalt und/oder Nickel enthalten, umsetzt.

Aus den bei dieser Herstellungsweise erhaltenen Reaktionsgemischen, die u.a. Pyrrol und Pyrrolidine enthalten, läßt sich das Pyrrol durch das vorliegende Aufarbeitungsverfahren besonders günstig gewinnen.

Die jeweiligen Anteile an Pyrrolen bzw. Pyrrolidinen wurden gaschromatographisch mit einem FID als Detektor bestimmt. Der Pyrrolgehalt der Rohlösung wurde nach Eichung mit innerem Standort bestimmt. Der Wassergehalt wurde nach Fischer ermittelt.

### Beispiele

Bei allen im folgenden beschriebenen Beispielen kam eine diskontinuierliche, ölbadbeheizte, 1 Liter-Vakuum-Rektifikationsapparatur zum Einsatz. Die verspiegelte 30 mm Vakuummantel-Glaskolonne war 300 mm lang und war mit Edelstahl-Netzdrahtwendeln (Ø 3 mm) gefüllt. Der Rücklauf konnte über einen Wasserauskreiser gefahren werden. Bei allen Beispielen wurde mit einem Rücklaufverhältnis von 1:1 gearbeitet.

### Beispiel 1 (Gewinnung eines Vordestillats)

500 g Rohpyrrol, das durch Dehydrieren von Pyrrolidin bis 275°C an einem Palladiumträgerkatalysator (0,5 Gew.-% Palladium, 1,0 Gew.-% Mangan, 5,0 Gew.-% Cer-(IV)-oxid auf Aluminiumoxid, Korngröße 0,2 bis 0,6 mm) hergestellt war, wurden rektifiziert. Laut GC-Analyse enthielt das Rohpyrrol 72 % Pyrrol, 14 % Pyrrolidin, und 1 % Leichtersieder (alkylsubstituierte Pyrrole und Pyrrolidine, Angaben in Gew.-%). Nach Karl-Fischer lag der Wassergehalt bei 4 %. Die erste Fraktion wurde bei konstant 150 mbar, die zweite bei 150 - 110 mbar und die dritte bei 110 - 40 mbar gewonnen. Die Sumpftemperatur stieg von anfangs 80°C bis 110°C. Aus der ersten Fraktion schieden sich 8 g einer Wasserphase ab. Die organische Phase (40 g, Übergang bis 79°C) enthielt 65 % Pyrrol, 10 % Pyrrolidin, 12 % Wasser und 13 % Leichtsieder. Die zweite Fraktion (285 g, Kopftemperatur 79 - 80°C) enthielt 81 % Pyrrol, 16 % Pyrrolidin und 3 % Wasser. Die dritte Fraktion (100 g, Übergang 80 - 54°C) enthielt 86 % Pyrrol und 14 % Pyrrolidin. Über alle Fraktionen gerechnet konnten 95 % des eingesetzten Pyrrols gewonnen werden.

Die Fraktionen 2 und 3 wurden vereinigt und in den Beispielen 2 - 5 als "Vordestillat" eingesetzt.

### Beispiel 2

500 g Vordestillat wurden mit 113 g 50%-iger Schwefelsäure und 84 g Wasser versetzt. 203 g wäßrige Phase wurden abgetrennt. Die organische Phase wurde rektifiziert. Es wurde zunächst bei 200 mbar Vakuum gearbeitet. Nach 63 g Vorlauf mit 78 % Pyrrol wurde die erste Hauptfraktion (200 g, Übergang 84°C) mit > 97 % Pyrrol, 2 % Wasser und <0,1 % Pyrrolidin gewonnen. Im Verlauf der zweiten Hauptfraktion (100 g) wurde der Kolonnendruck auf 40 mbar gesenkt. Die Sumpftemperatur wurde unter 100°C gehalten. Die Analysen ergaben > 99 % Pyrrol, 0,6 % Wasser und < 0,1 % Pyrrolidin. Die Destillation wurde bei einer Sumpftemperatur von 110°C abgebrochen. Die letzte Fraktion (33 g) enthielt 0,3 % Pyrrolidin und > 99 % Pyrrol. Bilanziert man über alle Fraktionen, so wurden 95 % des eingesetzten Pyrrols überdestilliert.

### Beispiel 3

500 g Vordestillat wurden mit 59 g Ameisensäure auf pH 7 eingestellt. Bei Normaldruck wurden 52 g Wasser ausgekreist. Nach einem Vorlauf von 60 g wurde bei 85 mbar eine Hauptfraktion (200 g, Kopftemperatur 65°C) mit einem Pyrrolgehalt von > 99 % gewonnen. Der Wasser- wie der Pyrrolidingehalt der Hauptfraktionen lagen jeweils < 0,1 %.

### Beispiel 4

500 g Vordestillat wurden mit 225 g einer 25%-igen methanolischen Schwefelsäure versetzt. Methanol wurde bei Normaldruck abdestilliert. Der Druck wurde auf 85 mbar reduziert. Nach 60 g Vorlauf wurde eine Hauptfraktion von 200 g genommen (85°C Kopftemperatur). Der Pyrrolidingehalt lag bei < 0,1 %.

### Beispiel 5

500 g Vordestillat wurden unter Wasserkühlung mit 70 g Methylformiat versetzt. Es wurde 6 h bei Normaldruck am Rückfluß erhitzt. Überschüssiges Methylformiat ging dabei ins Abgas. Es wurde bei 85 mbar rektifiziert. Nach 60 g Vorlauf gewann man eine Hauptfraktion von 200 g (850C Kopftemperatur) mit einem Wasser- wie einem Pyrrolidingehalt < 0,1 %.

### Beispiel 6 (ohne Vordestillation)

Aus 500 g Rohpyrrol wurden mit 120 g Cyclohexan 16 g Wasser ausgekreist. Das Cyclohexan wurde bei 350 mbar abgezogen. Anschließend wurden 69 g Methylformiat zugegeben. Die Lösung wurde bei 40 mbar Vakuum und einer maximalen Sumpftemperatur von 160°C ausdestilliert. 97 % des eingesetzten Pyrrols gingen pyrrolidinfrei (<0,1 %) über.

Es stellte sich somit überraschend heraus, daß auch ohne Vordestillation hochreines Pyrrol erhalten werden konnte.

## Patentansprüche

1. Verfahren zur Reinigung von bei der Herstellung von Pyrrolen der allgemeinen Formel I 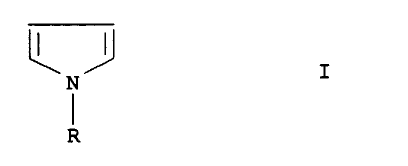 in der R Wasserstoff oder einen C₁- bis C₆-Alkylrest bedeutet, anfallenden Rohpyrrolen, dadurch gekennzeichnet, daß man die Mischung, die das Rohpyrrol enthält, mit einer Säure oder wenn R Wasserstoff bedeutet, mit einer Säure oder einem aktivierten Carbonsäurederivat versetzt und das Pyrrol aus der Mischung bei Unterdruck und einer Sumpftemperatur bis zu 160°C destillativ abtrennt.

2. Verfahren nach Anspruch 1, dadurch gekennzeichnet, daß man die Rohpyrrol enthaltende Mischung mit einer mindestens 10 Gew.-%igen Mineral- oder Carbonsäure versetzt und das Pyrrol aus der Mischung bei 300 bis 20 mbar und einer Sumpftemperatur von 60 bis 90°C destillativ abtrennt.

3. Verfahren nach Anspruch 2, dadurch gekennzeichnet, daß man als Mineralsäure Schwefelsäure einsetzt.

4. Verfahren nach Anspruch 2, dadurch gekennzeichnet, daß man die Rohpyrrol enthaltende Mischung mit einer 10 bis 30 Gew.-%igen Mineral- oder Carbonsäure versetzt und die sich bildende wäßrige Phase vor der destillativen Aufarbeitung des Pyrrols abtrennt.

5. Verfahren nach Anspruch 2, dadurch gekennzeichnet, daß man als Carbonsäure eine 60 bis 95 Gew.-%ige Ameisensäure einsetzt.

6. Verfahren nach Anspruch 1, dadurch gekennzeichnet, daß man die Rohpyrrol enthaltende Mischung unter Erwärmen oder Kühlen mit einem aktivierten Carbonsäurederivat versetzt und das Pyrrol aus der Mischung bei Drücken von 1000 bis 200 mbar und Sumpftemperaturen von 80 bis 160°C destillativ abtrennt.

7. Verfahren nach Anspruch 6, dadurch gekennzeichnet, daß man als aktivierte Carbonsäurederivate Methyl- oder Ethylester von C₁- bis C₁-C₃-Monocarbonsäuren einsetzt.

8. Verfahren nach Anspruch 1, dadurch gekennzeichnet, daß man die zu reinigenden Rohpyrrole dadurch herstellt, daß man Pyrrolidine der Formel II 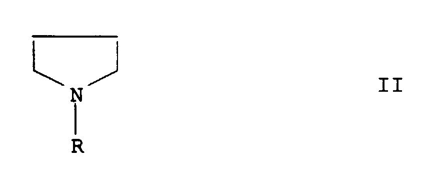 worin R die im Anspruch 1 angegebene Bedeutung hat, bei Temperaturen von 160 bis 400°C in Gegenwart von Palladiumträgerkatalysatoren, die basische Verbindungen der Alkali-, Erdalkalimetalle und/oder Metalle der seltenen Erden und/oder Elemente der Gruppen Ib, IIb, VIIb, Cobalt und/oder Nickel enthalten, umsetzt.

9. Verfahren nach Anspruch 1, dadurch gekennzeichnet, daß man die Mischung, die das Rohpyrrol enthält, vor der Umsetzung mit der Säure oder dem aktivierten Carbonsäurederivat einer Vordestillation unterwirft.

## Claims

1. A process for purifying crude pyrroles obtained in the preparation of pyrroles of the general formula I 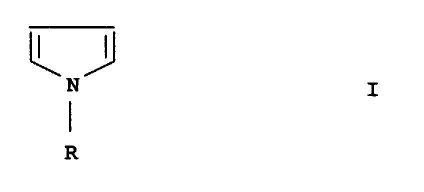 where R is hydrogen or a C₁- to C₆-alkyl radical, which comprises treating the mixture containing the crude pyrrole with an acid or, if R is hydrogen, with an acid or an activated carboxylic acid derivative and removing the pyrrole from the mixture by distillation at reduced pressure and a bottom temperature of up to 160°C.

2. A process as claimed in claim 1, wherein the mixture containing the crude pyrrole is treated with an at least 10% strength by weight mineral or carboxylic acid and the pyrrole is removed from the mixture by distillation at 300 to 20 mbar and a bottom temperature from 60 to 90°C.

3. A process as claimed in claim 2, wherein the mineral acid employed is sulfuric acid.

4. A process as claimed in claim 2, wherein the mixture containing the crude pyrrole is treated with a 10 to 30% strength by weight mineral or carboxylic acid and the aqueous phase formed is removed before the distillative work-up of the pyrrole.

5. A process as claimed in claim 2, wherein the carboxylic acid employed is a 60 to 95% strength by weight formic acid.

6. A process as claimed in claim 1, wherein the mixture containing the crude pyrrole is treated with an activated carboxylic acid derivative with heating or cooling and the pyrrole is removed from the mixture by distillation at pressures from 1000 to 200 mbar and bottom temperatures from 80 to 160°C.

7. A process as claimed in claim 6, wherein the activated carboxylic acid derivatives employed are methyl or ethyl esters of C₁- to C₃-monocarboxylic acids.

8. A process as claimed in claim 1, wherein the crude pyrroles to be purified are prepared by reacting pyrrolidines of the formula II 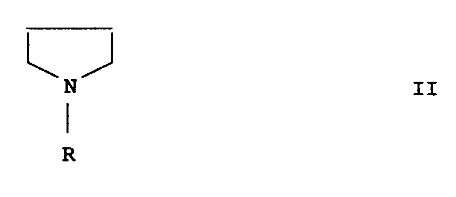 where R has the meaning indicated in claim 1, at from 160 to 400°C in the presence of supported palladium catalysts which contain basic compounds of the alkali metals or alkaline earth metals and/or rare earth metals and/or elements of the groups Ib, IIb, VIIb, cobalt and/or nickel.

9. A process as claimed in claim 1, wherein the mixture which contains the crude pyrrole is subjected to a predistillation before the reaction with the acid or the activated carboxylic acid derivative.

## Revendications

1. Procédé pour purifier les pyrroles bruts obtenus lors de la préparation de pyrroles de formule générale I 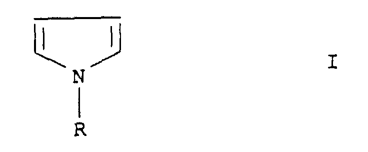 dans laquelle R est un atome d'hydrogène ou un radical alkyle en C₁-C₆, caractérisé en ce qu'on ajoute un acide au mélange contenant le pyrrole brut, ou encore, quand R est un atome d'hydrogène, on lui ajoute un acide ou un dérivé activé d'un acide carboxylique, et on sépare le pyrrole du mélange par distillation sous basse pression, à une température de fond allant jusqu'à 160°C.

2. Procédé selon la revendication 1, caractérisé en ce qu'on ajoute au mélange contenant du pyrrole brut un acide minéral ou carboxylique à au moins 10 %, et on sépare le pyrrole du mélange par distillation sous une pression de 300 à 20 mbar et à une température de fond de 60 à 90°C.

3. Procédé selon la revendication 2, caractérisé en ce qu'on utilise en tant qu'acide minéral l'acide sulfurique.

4. Procédé selon la revendication 2, caractérisé en ce qu'on ajoute au mélange contenant le pyrrole brut un acide minéral ou carboxylique à 10 à 30 %, et la phase aqueuse qui se forme est séparée avant le traitement du pyrrole par distillation.

5. Procédé selon la revendication 2, caractérisé en ce qu'on utilise en tant qu'acide carboxylique un acide formique à 60 à 95 %.

6. Procédé selon la revendication 1, caractérisé en ce qu'on ajoute au mélange contenant le pyrrole brut, tout en chauffant ou en refroidissant, un dérivé activé d'acide carboxylique, et on sépare le pyrrole du mélange par distillation sous des pressions de 1000 à 200 mbar et à des températures de fond de 80 à 160°C.

7. Procédé selon la revendication 6, caractérisé en ce qu'on utilise en tant que dérivés activés d'acide carboxylique l'ester méthylique ou éthylique d'acides monocarboxyliques en C₁-C₃.

8. Procédé selon la revendication 1, caractérisé en ce qu'on prépare les pyrroles bruts à purifier en faisant réagir des pyrrolidines de formule II 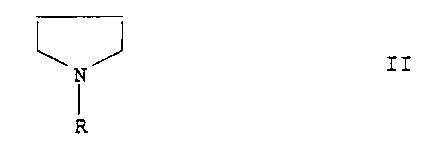 dans laquelle R a les significations données dans la revendication 1, à des températures de 160 à 400°C, en présence de catalyseurs au palladium supporté, qui contiennent des composés basiques des métaux alcalins ou alcalino-terreux et/ou des métaux des terres rares et/ou des éléments des groupes Ib, IIb, VIIb, du cobalt et/ou du nickel.

9. Procédé selon la revendication 1, caractérisé en ce qu'on soumet à une distillation préalable le mélange contenant le pyrrole brut, avant réaction avec l'acide ou le dérivé activé d'acide carboxylique.
